(19) **Europäisches Patentamt**
**European Patent Office**
**Office européen des brevets**

(11) **EP 1 967 134 B1**

(12) **EUROPEAN PATENT SPECIFICATION**

(45) Date of publication and mention
of the grant of the patent:
**17.11.2010   Bulletin 2010/46**

(51) Int Cl.:
***A61B 5/0275*** *(2006.01)*

(21) Application number: **07123550.1**

(22) Date of filing: **19.12.2007**

(54) **Apparatus for measuring perfusion rate of legs**

Vorrichtung zum Messen der Perfusionsrate an Beinen

Appareil de mesure du taux de perfusion de jambes

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **09.03.2007   KR 20070023496**

(43) Date of publication of application:
**10.09.2008   Bulletin 2008/37**

(73) Proprietor: **Korea Advanced Institute of Science and Technology**
**305-701 Daejeon (KR)**

(72) Inventors:
• **Choi, Chulhee**
  **305-701, Daejeon (KR)**
• **Kang, Yujung**
  **305-701 Daejeon (KR)**
• **Choi, Myunghwan**
  **305-701 Daejeon (KR)**

(74) Representative: **Office Freylinger**
  **P.O. Box 48**
  **8001 Strassen (LU)**

(56) References cited:
**EP-A- 0 059 032        WO-A-01/22870**
**DE-A1- 19 838 606      DE-C1- 10 059 070**

**Description**

BACKGROUND OF THE INVENTION

1. Field of the Invention

The present invention relates, in general, to an apparatus for measuring the perfusion rate of legs, and, more particularly, to an apparatus for measuring the perfusion rate of legs, which includes a leg support unit capable of designating the legs of a living body as a fluorescence imaging region using Indocyanine Green (ICG) and then supporting the legs, so that the probability of erroneous measurement, attributable to the movement of the living body, can be minimized at the time of continuously capturing images for a predetermined period of time, and which includes a light leakage prevention unit indicating a darkroom structure for intercepting light other than light emitted from a light source required for measurement, so that obstructions to the measurement of fluorescence images can be eliminated, the functional characteristics of the perfusion rate can be measured with high spatial sensitivity, and the functional measurement of the perfusion rate in a specific region can be performed.

2. Description of the Related Art

Generally, angiography is technology for measuring vascular structure by acquiring the dispersion and emission of light, caused by the intrinsic optical property of the blood or the administrated contrast medium in intravascular space, using a light source capable of imaging the contrast medium, thus measuring the perfusion of tissue.
Such angiography includes laser Doppler imaging, which measures the degree of dispersion of laser light using the blood flow velocity at the skin, X-ray angiography, which measures the inner opening of blood vessels by capturing images through an X-ray using a contrast medium for blood vessels, and ICG videoangiography, based on Indocyanine Green (ICG), which measures sequential changes of the concentration of the ICG, which is a contrast medium, in the blood, using the ICG at the near infrared ray.
However, laser Doppler imaging is problematic in that, when the blood flow velocity is decreased, sensitivity is decreased. X-ray angiography is problematic in that, since a structural image indicating the structure of the inner opening of blood vessels, rather than actual blood flow, is obtained, the perfusion rate of tissue cannot be measured. ICG videoangiography has several problems, in that there is a probability that a fluorescence images, measured by radiating a near infrared ray, will be erroneously measured due to an external light source, and in that the functional characteristics of the perfusion rate cannot be quantitatively calculated.
DE 100 59 070 C1 describes a device for the determination of the perfusion rate in a patient and the use of it during surgical operations using a infrared laser and a CCD camera to detect a fluorescence signal light emitted by Indocyanine Green (ICG) injected previously to the patient.
WO01/22870 describes a device with a laser for exciting the fluorescent imaging agent to emit radiation of a particular wavelength, a CCD camera with a band pass filter for capturing the emissions from the imaging agent, and a distance sensor incorporating a visual display for providing feedback to the physician saying that the laser be located at an optimal distance from the vessel of interest for the capture of high quality images.

SUMMARY OF THE INVENTION

Accordingly, the present invention has been made keeping in mind the above problems occurring in the prior art, and an object of the present invention is to provide an apparatus for measuring the perfusion rate of legs, which includes a leg support unit capable of designating the legs of a living body as a fluorescence imaging region using Indocyanine Green (ICG), and then supporting the legs, so that the probability of erroneous measurement, attributable to the movement of the living body, can be minimized at the time of continuously capturing images for a predetermined period of time, and which includes a light leakage prevention unit, indicating a darkroom structure for intercepting light other than light emitted from a light source required for measurement, so that obstructions to the measurement of fluorescence images can be eliminated, the functional characteristics of the perfusion rate can be measured with high spatial sensitivity, and the functional measurement of the perfusion rate in a specific region can be performed.
In order to accomplish the above object, the present invention provides an apparatus for measuring a perfusion rate of legs, comprising a light leakage prevention unit including a light emitting device for radiating light having a certain wavelength onto a living body injected with Indocyanine Green (ICG), and a light leakage prevention housing formed to prevent transmission of external light; a data acquisition unit including a band pass filter for passing therethrough only light, having a near infrared wavelength, in a fluorescence image emitted by the ICG and the light emitting device, and a Charge Coupled Device (CCD) camera for photographing a near infrared wavelength region; a data processing unit for calculating a perfusion rate of the living body on a basis of data about the near infrared wavelength region through a data cable connected to the data acquisition unit; and a data output unit for outputting the perfusion rate calculated by the data processing unit.

The light leakage prevention housing includes a leg support unit, which is a structure capable of supporting legs of the living body.

Preferably, the leg support unit may include a leg support having a certain width and a certain height, required to support both legs of the living body; and a footrest implemented to allow feet of the living body to be placed thereon.

Preferably, the light emitting device may be implemented using any one of a laser having a wavelength from 750 to 780 nm, a white light source, and a light emitting diode, each having a band pass filter corresponding to a region of the wavelength.

Preferably, the light emitting device may be implemented using one or more light emitting devices, and be constructed to be capable of adjusting an intensity of light emitted therefrom.

Preferably, the data processing unit may include digitization means for processing data input through the data cable to have fluorescence intensities for respective regions over time in a region of interest; determination means for recognizing ischemic patterns of respective regions on a basis of the fluorescence intensities over time, and calculating correlation coefficients between the ischemic patterns and adjacent regions; and perfusion rate calculation means for calculating the perfusion rate using data input from the determination means.

Preferably, the data output unit may assign predefined colors depending on the perfusion rate and may color respective regions in the assigned colors, thus visually outputting the perfusion rate in a form of an image.

BRIEF DESCRIPTION OF THE DRAWINGS

The above and other objects, features and other advantages of the present invention will be more clearly understood from the following detailed description taken in conjunction with the accompanying drawings, in which:

FIG. 1 is a block diagram schematically showing an apparatus for measuring the perfusion rate of legs according to the present invention;

FIG. 2 is a perspective view schematically showing an application example of an apparatus for measuring the perfusion rate of legs according to an embodiment of the present invention;

FIG. 3 is a perspective view schematically showing another application example of the apparatus for measuring the perfusion rate of legs according to the embodiment of the present invention;

FIGS. 4A and 4B are perspective views schematically showing a further application example of the apparatus for measuring the perfusion rate of legs according to the embodiment of the present invention; and

FIG. 5 is a flowchart schematically showing a method of measuring the perfusion rate of legs according to the present invention.

DESCRIPTION OF THE PREFERRED EMBODIMENTS

Hereinafter, embodiments of the present invention will be described in detail with reference to the attached drawings.

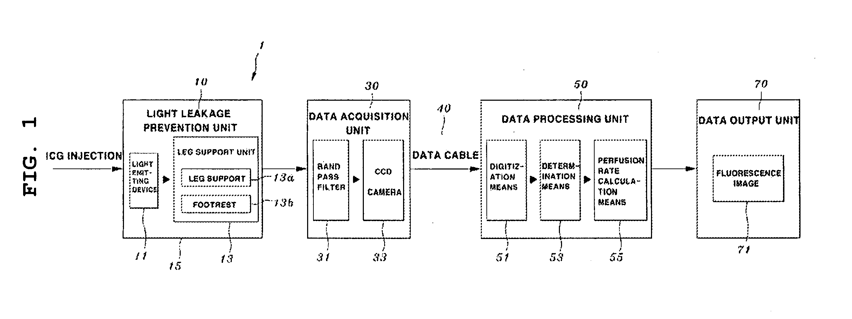

FIG. 1 is a block diagram schematically showing an apparatus for measuring the perfusion rate of legs according to an embodiment of the present invention. As shown in the drawing, an apparatus 1 for measuring the perfusion rate of legs according to the present invention includes a light leakage prevention unit 10, a data acquisition unit 30, a data cable 40, a data processing unit 50, and a data output unit 70.

In this case, the light leakage prevention unit 10 includes a light emitting device 11, a leg support unit 13, and a light leakage prevention housing 15.

The light emitting device 11 is provided to emit light having a certain wavelength to a living body injected with Indocyanine Green (ICG), and is operated such that light from the light emitting device 11 is radiated onto the living body, so that the ICG in the tissue of the living body, injected with the ICG, is activated, and thus a fluorescence signal obtained from the tissue can be observed.

Here, the light emitted from the light emitting device 11 has a wavelength from 750 to 780 nm, and is radiated onto the legs of the living body. The near infrared ray having that wavelength is radiated to observe fluorescence caused by the injection of the ICG. A light emitting diode or a laser having the above wavelength can be used as a light source for emitting light. In order to allow white light having that wavelength to be radiated as the near infrared ray, a Band Pass Filter (BPF) can be used as a filter. In this case, one or more BPFs can be used, the locations thereof can be adjusted, and the intensity of the near infrared ray having the wavelength can be adjusted.

Further, the leg support unit 13 is formed to allow both legs of the living body to be held so that images can be continuously acquired as the concentration of the ICG decreases. The leg support unit 13 is modified to make it possible to photograph the legs in all cases where the living body sits, lies on his or her back, or lies on his or her side.

The leg support unit 13 includes a leg support 13a having a certain width and a certain height to support both legs of the living body, and a footrest 13b on which the feet of the living body are placed.

The leg support 13a can be formed to have a certain height, required to support a portion of each leg below the joint of a knee, and the footrest 13b can be formed in the shape of a curve to correspond to the curve of the bottom surfaces of the feet. The legs of the living body can be held while forming a certain angle with respect to the bottom surface of the leg support 13a.

Moreover, the shape of the leg support 13a can be changed to variously support the portions of the legs below the knees in the state in which the living body lies on his or her back, or lies on his or her side.

Further, the light leakage prevention housing 15 is formed to prevent the transmission of external light, and thus functions as a kind of darkroom. The light leakage prevention housing 15 is provided to enclose the light emitting device 11 for radiating light onto the legs of the living body, the leg support unit 13, and the legs of the living body, and is formed to enclose the components, such as the light emitting device 11 and the leg support unit 13, while limiting the transmission of light, regardless of whether the living body sits, lies on his or her back, or lies on his or her side. The structure of the light leakage prevention housing 15 can be modified.

Further, the data acquisition unit 30 includes a band pass filter 31 and a Charge Coupled Device (CCD) camera 33.

The band pass filter 31 is configured to pass only light having a specific wavelength therethrough so as to receive a fluorescence signal generated from the living body due to the light emitted from the light emitting device 11, and is provided, in detail, to pass therethrough only a near infrared ray, having a wavelength from 800 to 850 nm, in the fluorescence signal output from the living body due to the light.

Further, the CCD camera 33 senses the light passing through the band pass filter 31, and converts the light into a digital signal. The CCD camera, which is one kind of digital camera, converts an image into an electrical signal using a CCD, and thus stores an analog image in a storage medium as digital data.

In this case, the CCD camera 33 captures the fluorescence signal, which has been input to the data acquisition unit 30 and has passed through the band pass filter 31, and transmits the captured signal through the data cable 40 to perform image processing in the form of digital data.

Furthermore, the data acquisition unit 30 and the light emitting device 11 can be placed adjacent to each other. The data acquisition unit 30 is located within the light leakage prevention housing 15 of the light leakage prevention unit 10 to photograph the legs of the living body in the housing.

The data processing unit 50 includes a digitization means 51, a determination means 53, and a perfusion rate calculation means 55, and preferably further includes an input means (not shown) for receiving digital data from the data acquisition unit 30, and an output means (not shown) for outputting the digital data to the data output unit 70.

Here, the digital data output from the data acquisition unit 30 is transmitted to the data processing unit 50 through the data cable 40. Preferably, the data cable 40 can be implemented using an RS-232C cable, a parallel port, an IEEE 1934 cable, a Universal Serial Bus (USB), etc.

The digitization means 51 is provided to process an input signal to have fluorescence intensities over time in a region of interest. The determination means 53 calculates correlation coefficients between perfusion patterns for respective regions and surrounding signals on the basis of the processed fluorescence intensity over time. The calculation means 55 calculates a perfusion rate using the data input from the determination means 53.

The above-described data processing unit 50 measures the fluorescence intensities of the ICG for respective regions as time elapses after the ICG is injected into the living body, analyses patterns for respective regions, and also analyzes correlation coefficients between the patterns and the surrounding signals.

The analysis of the patterns for respective regions is represented by the following Equation.

The degree of variation in the fluorescence intensity of the ICG relative to time in ischemic tissue, the perfusion rate of which is lower than that of normal tissue, can be represented by the following Equation [1] on the assumption that ICG fluorescent particles (FLnor) flow into normal tissue at the perfusion rate at which ischemic tissue is perfused and ICG fluorescent particles (FLisc) flow from the ischemic tissue at the same perfusion rate.

$$FLisc = \frac{PA}{P - 1/\tau}(e^{\frac{-t}{\tau}} - e^{-Ft}) \qquad [1]$$

In this case, Tmax is used as a value for the efficient recognition of an ischemic pattern.

Further, Tmax at which the fluorescence intensity of the ICG in the ischemic tissue is maximized is the position at which the value differentiated with respect to time becomes 0, and thus the following Equation can be calculated.

$$-\ln \tau - \frac{T_{\max}}{\tau} = \ln P - P T_{\max} \qquad [2]$$

The above method of analyzing the correlation coefficient derives a correlation coefficient between each region and a region adjacent thereto versus time using the following Equation.

$$\rho_{X.Y} = \frac{\mathrm{cov}(X,Y)}{\sigma_X \sigma_Y} = \frac{E((X-\mu_X)(Y-\mu_Y))}{\sigma_X \sigma_Y} \qquad [3]$$

The perfusion rate of tissue can be finally derived from the values obtained through the above two equations.
The perfusion rate calculated in this way is transmitted to the data output unit 70, so that predefined colors are assigned depending on the perfusion rate, and respective regions are colored, and thus the perfusion rate can be visually displayed in the form of an image.
Further, when a Region of Interest (ROI), the perfusion rate of which is desired to be measured, is designated in the living body, the fluorescence intensities of the ICG over time are automatically digitized from continuous ICG image data, and the digitized results are processed as temporal or spatial information.
As described above, in order to measure ICG dynamics in a living body using the apparatus 1 for measuring the perfusion rate of legs according to the present invention, the ICG is injected using intravenous injection, and variation in the concentration of the ICG over time in tissue is measured using the above method. At this time, the variation in the concentration of the ICG in the tissue can be measured by directly obtaining a near infrared image of the tissue, or using a spectroscopic technique.

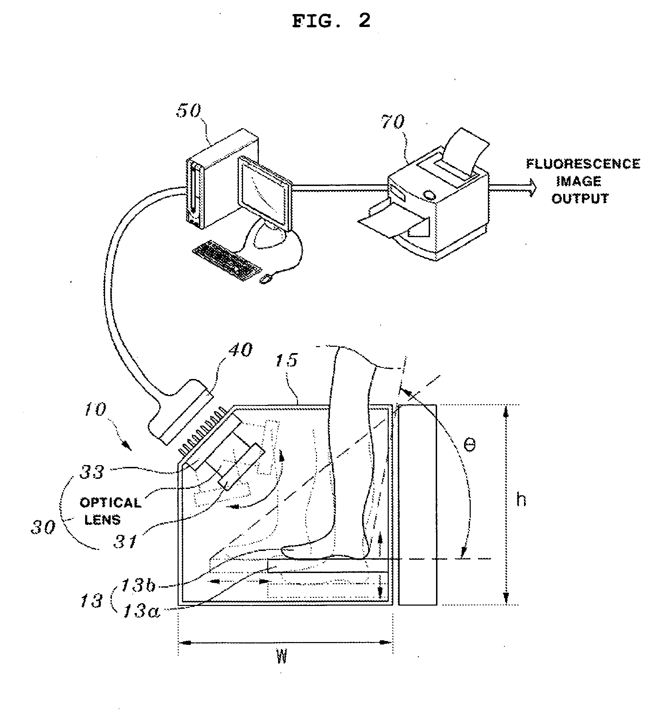

FIG. 2 is a perspective view schematically showing an application example of the apparatus for measuring the perfusion rate of legs according to the embodiment of the present invention. As shown in FIG. 2, in order to measure the perfusion rate of a region of interest when a living body sits, portions of both legs ranging up to predetermined locations of knees are inserted into the light leakage prevention housing 15, and the location of the horizontal portion of the leg support 13a is vertically changed along upward and downward directions to adjust the height h thereof, and thus a portion of an image sensed by the CCD camera 33 of the data acquisition unit 30 can be adjusted.
Through the above method, the location of the horizontal portion of the leg support 13a is horizontally changed along forward and backward directions to adjust an angle θ, and thus a portion of an image sensed by the CCD camera 33 of the data acquisition unit 30 can be adjusted. The location of the leg support 13a can be preferably adjusted along forward and backward directions.
The CCD camera 33 of the data acquisition unit 30 is moved and placed upwards and downwards, left and right, or forwards and backwards, so that the region of an image desired to be acquired can be adjusted depending on the region of interest.
Moreover, the footrest 13b is preferably constructed to move forwards and backwards, or left and right.
Further, analog data about the fluorescence intensities input from the data acquisition unit 30 is transmitted to the data processing unit 50 as digital data through the data cable 40. A perfusion rate is calculated on the basis of the digital data using a preprogrammed operation, and respective regions are colored in the colors based on the perfusion rate, and thus a fluorescent image is output to the data output unit 70, including a monitor, a printer, etc.

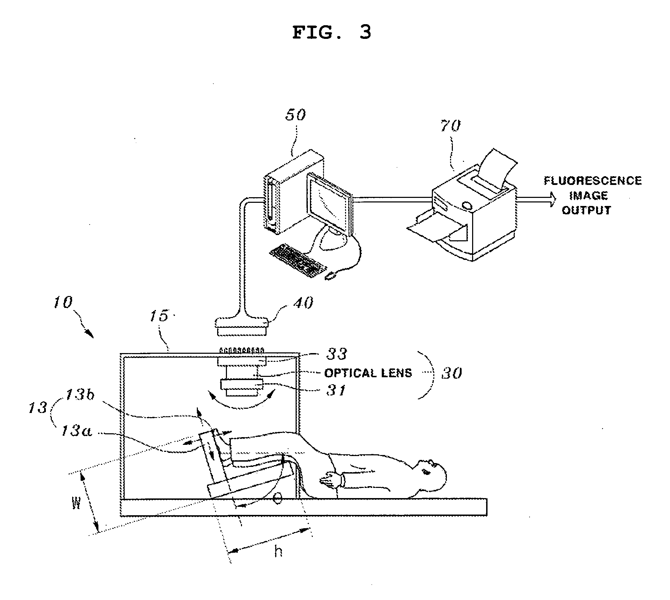

FIG. 3 is a perspective view schematically showing another application example of the apparatus for measuring the perfusion rate of legs according to the embodiment of the present invention. As shown in FIG. 3, in order to measure the perfusion rate of a region of interest when the living body lies on his or her back, portions of both legs ranging up to predetermined locations of knees or predetermined locations of thighs are inserted into the light leakage prevention housing 15 to measure the fluorescence intensities of the region of interest, and the location of the horizontal portion of the leg support 13a is vertically changed along upward and downward directions to adjust the height h thereof, and thus a portion of an image sensed by the CCD camera 33 of the data acquisition unit 30 can be adjusted.
Through the above method, the location of the horizontal portion of the leg support 13a is horizontally changed along forward and backward directions to adjust an angle θ, and thus a portion of an image sensed by the CCD camera 33 of the data acquisition unit 30 can be adjusted. The location of the leg support 13a can be preferably adjusted along forward and backward directions.
The CCD camera 33 of the data acquisition unit 30 is moved and placed upwards and downwards, left and right, or forwards and backwards, so that the region of an image desired to be acquired can be adjusted depending on a region of interest.
Moreover, the footrest 13b is preferably constructed to move forwards and backwards, or left and right.

Further, analog data about the fluorescence intensities input from the data acquisition unit 30 is transmitted to the data processing unit 50 as digital data through the data cable 40. A perfusion rate is calculated on the basis of the digital data using a preprogrammed operation, and respective regions are colored in the colors based on the perfusion rate, and thus a fluorescent image is output to the data output unit 70, including a monitor, a printer, etc.

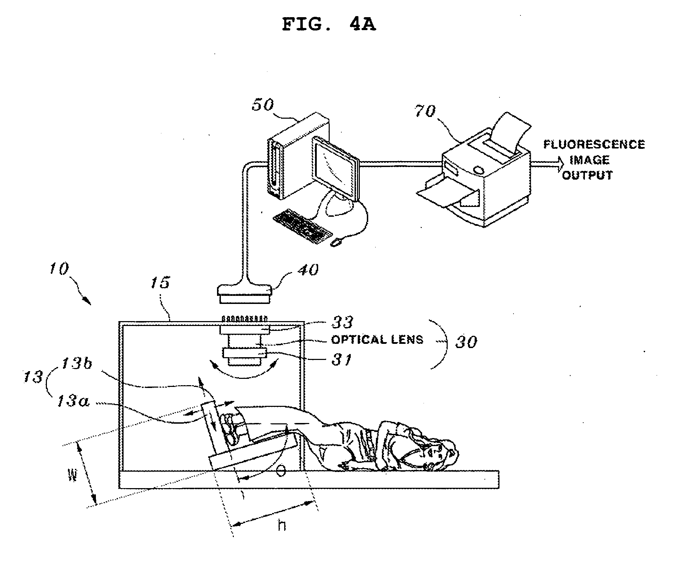

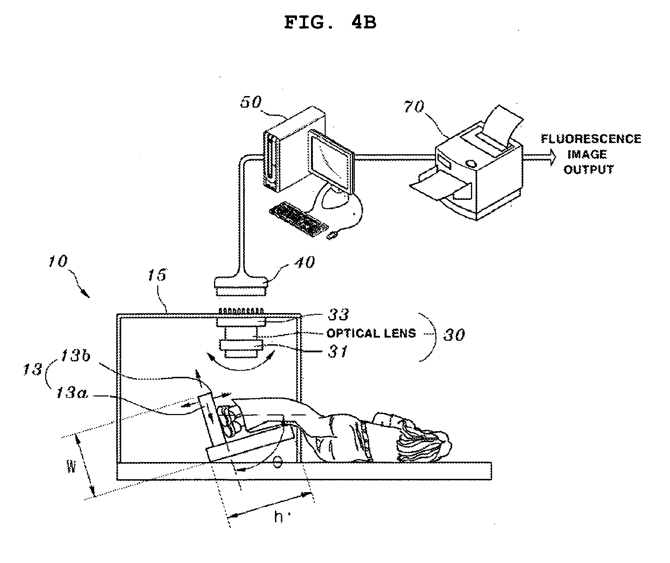

FIGS. 4A and 4B are perspective views schematically showing a further application example of the apparatus for measuring the perfusion rate of legs according to the embodiment of the present invention. As shown in the drawings, FIGS. 4A and 4B show that, in order to measure the perfusion rate of a region of interest when the living body lies on his or her left and right sides, respectively, portions of both legs ranging up to predetermined locations of knees or predetermined locations of thighs are inserted into the light leakage prevention housing 15 to measure the fluorescence intensities of the region of interest, and the location of the horizontal portion of the leg support 13a is vertically changed along upward and downward directions to adjust the height h thereof, and thus a portion of an image sensed by the CCD camera 33 of the data acquisition unit 30 can be adjusted.

Through the above method, the location of the horizontal portion of the leg support 13a is horizontally changed along forward and backward directions to adjust an angle θ, and thus a portion of an image sensed by the CCD camera 33 of the data acquisition unit 30 can be adjusted. The location of the leg support 13a can be preferably adjusted along forward and backward directions.

The CCD camera 33 of the data acquisition unit 30 is moved and placed upwards and downwards, left and right, or forwards and backwards, so that the region of an image to be acquired can be adjusted depending on a region of interest.

Moreover, the footrest 13b is preferably constructed to move forwards and backwards or left and right.

Further, analog data about the fluorescence intensities input from the data acquisition unit 30 is transmitted to the data processing unit 50 as digital data through the data cable 40. A perfusion rate is calculated on the basis of the digital data using a preprogrammed operation, and respective regions are colored in the colors based on the perfusion rate, and thus a fluorescent image is output to the data output unit 70, including a monitor, a printer, etc.

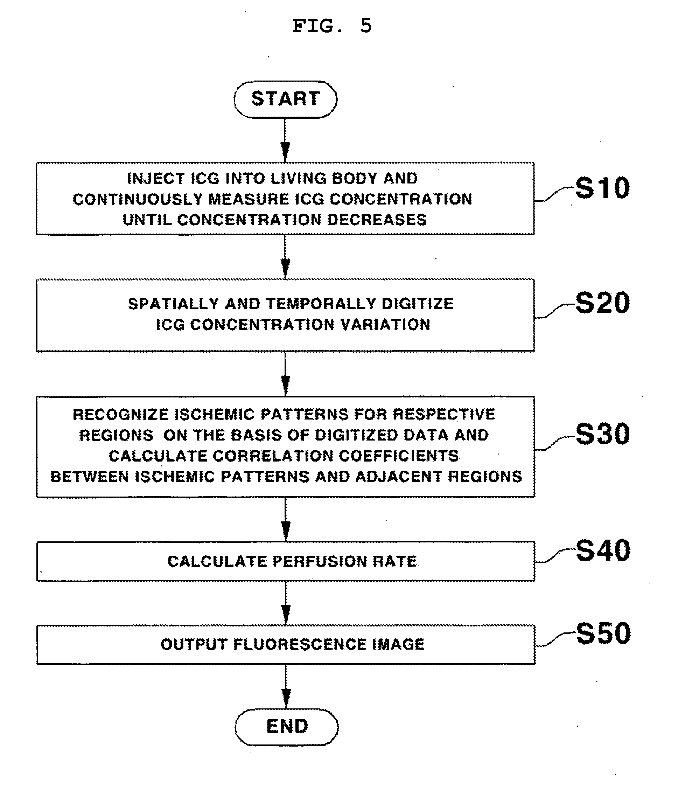

FIG. 5 is a flowchart schematically showing a measurement method using the apparatus for measuring the perfusion rate of legs according to the present invention. As shown in the drawing, the measurement method using the apparatus for measuring the perfusion rate of legs according to the present invention is performed to inject ICG into a living body and to continuously measure the concentration of the ICG through the data acquisition unit until the concentration of the ICG decreases at step S10.

Further, variation in the concentration of the ICG is transmitted to the data processing unit through the data cable, and is analyzed and digitized, and the dynamics of the ICG over time are displayed in the form of a graph at step S20.

Furthermore, an ischemic pattern, obtained at the time at which the concentration of the ICG is maximized, is recognized from the digitized data, and a correlation coefficient between the ischemic pattern and an adjacent region is calculated at step S30. A perfusion rate is calculated through a preprogrammed operation at step S40, and respective regions are colored in predefined colors corresponding to the perfusion rate, and thus a fluorescence image is output at step S50.

Although the preferred embodiments of the present invention have been disclosed for illustrative purposes, those skilled in the art will appreciate that various modifications, additions and substitutions are possible, without departing from the scope and spirit of the invention as disclosed in the accompanying claims. In detail, the present invention proposes an apparatus that includes a leg support unit capable of designating the legs of a living body as a fluorescence imaging region using Indocyanine Green (ICG) and then supporting the legs, so that the probability of erroneous measurement, attributable to the movement of the living body, can be minimized at the time of continuously capturing images for a predetermined period of time, and that includes a light leakage prevention unit, which is a darkroom structure for intercepting light other than light emitted from a light source required for measurement, so that obstructions to the measurement of fluorescence images can be eliminated, the functional characteristics of the perfusion rate can be measured with high spatial sensitivity, and the functional measurement of the perfusion rate in a specific region can be performed.

As described above, the present invention having the above construction can realize several advantages in that a leg support unit, capable of designating the legs of a living body as a fluorescence imaging region using Indocyanine Green (ICG) and then supporting the legs, is provided, so that the probability of erroneous measurement, attributable to the movement of the living body, can be minimized at the time of continuously capturing images for a predetermined period of time, and in that a light leakage prevention unit, which is a darkroom structure for intercepting light other than light emitted from a light source required for measurement, is provided, so that obstructions to the measurement of fluorescence images can be eliminated, the functional characteristics of the perfusion rate can be measured with high spatial sensitivity, and the functional measurement of the perfusion rate in a specific region can be performed.

## Claims

1. An apparatus for measuring a perfusion rate of legs, comprising:

   a light leakage prevention unit (10) including a light emitting device (11) for radiating light having a certain wavelength onto a living body injected with Indocyanine Green (ICG), and a light leakage prevention housing (15) formed to prevent transmission of external light;
   a data acquisition unit (30) including a band pass filter (31) for passing therethrough only light, having a near infrared wavelength, in a fluorescence image emitted by the ICG and the light emitting device, and a Charge Coupled Device (CCD) camera (33) for photographing a near infrared wavelength region;
   a data processing unit (50) for calculating a perfusion rate of the living body on a basis of data about the near infrared wavelength region through a data cable connected to the data acquisition unit; and
   a data output unit (70) for outputting the perfusion rate calculated by the data processing unit,
   **characterized in that** the light leakage prevention housing (15) includes a leg support unit (13), which is a structure capable of supporting legs of the living body.

2. The apparatus according to claim 1, wherein the leg support unit (13) includes:

   a leg support (13a) having a certain width and a certain height, required to support both legs of the living body; and
   a footrest (13b) implemented to allow feet of the living body to be placed thereon.

3. The apparatus according to claim 1, wherein the light emitting device (11) is implemented using any one of a laser having a wavelength from 750 to 780 nm, a white light source, and a light emitting diode, each having a band pass filter corresponding to a region of the wavelength.

4. The apparatus according to claim 1, wherein the light emitting device (11) is implemented using one or more light emitting devices, and is constructed to be capable of adjusting an intensity of light emitted therefrom.

5. The apparatus according to claim 1, wherein the data processing unit (50) includes:

   digitization means (51) for processing data input through the data cable to have fluorescence intensities for respective regions over time in a region of interest;
   determination means (53) for recognizing ischemic patterns of respective regions on a basis of the fluorescence intensities over time, and calculating correlation coefficients between the ischemic patterns and adjacent regions; and
   perfusion rate calculation means (55) for calculating the perfusion rate using data input from the determination means.

6. The apparatus according to claim 5, wherein the data output unit (70) assigns predefined colors depending on the perfusion rate and colors respective regions in the assigned colors, thus visually outputting the perfusion rate in a form of an image.

## Patentansprüche

1. Vorrichtung zum Messen einer Perfusionsrate an Beien, aufweisend

   eine einen Lichtaustritt verhindernde Einheit (10), umfassend eine Lichtemissionseinrichtung (11) zum Ausstrahlen von Licht mit einer bestimmten Wellenlänge auf einen lebenden Körper, dem Indocyaningrün (ICG) gespritzt wurde, und ein einen Lichtaustritt verhinderndes Gehäuse (15), das so gebildet ist, dass es eine Übertragung von Außenlicht verhindert;
   eine Datenerfassungseinheit (30), umfassend ein Bandpassfilter (31) zum Hindurchführen nur von Licht, das eine Nah-Infrarot-Wellenlänge aufweist, in einem Fluoreszenzbild, das durch das ICG und die Lichtemissionseinrichtung ausgestrahlt wird, und eine ladungsgekoppelte (CCD-) Kamera (33) zum Fotografieren eines Nah-Infrarot-Wellenlängenbereichs;
   eine Datenverarbeitungseinheit (50) zum Berechnen einer Perfusionsrate des lebenden Körpers auf Grundlage von Daten über den Nah-Infrarot-Wellenlängenbereich durch eine an die Datenerfassungseinheit angeschlossene Datenleitung; und

eine Datenausgabeeinheit (70) zum Ausgeben der durch die Datenverarbeitungseinheit berechneten Perfusionsrate,

**dadurch gekennzeichnet, dass** das einen Lichtaustritt verhindernde Gehäuse (15) eine Beinauflageeinheit (13) umfasst, die eine Struktur ist, die in der Lage ist, Beine des lebenden Körpers zu stützen

2. Einrichtung nach Anspruch 1, wobei die Beinauflageeinheit (13) umfasst:

eine Beinauflage (13a) mit einer bestimmten Breite und einer bestimmten Höhe, die notwendig sind, um beide Beine des lebenden Körpers zu stützen; und
eine Fußstütze (13b), die so ausgeführt ist, dass Füße des lebenden Körpers auf dieser abgelegt werden können

3. Einrichtung nach Anspruch 1, wobei die Lichtemissionseinrichtung (11) ausgeführt ist unter Verwendung entweder eines Lasers mit einer Wellenlänge von 750 bis 780 nm, einer weißen Lichtquelle oder einer Leuchtdiode, die jeweils ein einem Bereich der Wellenlänge entsprechendes Bandpassfilter aufweisen

4. Einrichtung nach Anspruch 1, wobei die Lichtemissionseinrichtung (11) unter Verwendung von einer oder mehreren Lichtemissionseinrichtungen ausgeführt und so aufgebaut ist, dass sie in der Lage ist, eine Intensität von aus dieser ausgestrahltem Licht einzustellen

5. Einrichtung nach Anspruch 1, wobei die Datenverarbeitungseinheit (50) umfasst:

Digitalisierungsmittel (51) zum Verarbeiten von durch die Datenleitungeingegebenen Daten derart, dass sie Fluoreszenzintensitäten für jeweilige Bereiche im Zeitablauf in einem Bereich von Interesse aufweisen; Bestimmungsmittel (53) zum Erkennen von ischämischen Mustern von jeweiligen Bereichen auf Grundlage der Fluoreszenzintensitäten im Zeitablauf, und Berechnen von Korrelationskoeffzienten zwischen den ischämischen Mustern und angrenzenden Bereichen; und Mittel zum Berechnen der Perfusionsrate (55) zum Berechnen der Perfusionsrate unter Verwendung von durch die Bestimmungsmittel eingegebenen Daten

6. Einrichtung nach Anspruch 5, wobei die Datenausgabeeinheit (70) abhängig von der Perfusionsrate vorbestimmte Farben zuordnet und jeweilige Bereiche in den zugeordneten Farben färbt, wodurch die Perfusionsrate in Form eines Bildes visuell ausgegeben wird.

**Revendications**

1. Appareil de mesure de taux de perfusion de jambes, comprenant

une unité de prévention de fuite de lumière (10) incluant un dispositif d'émission de lumière (11) pour projeter une lumière ayant une certaine longueur d'onde sur un corps vivant ayant reçu par injection du vert d'indocyanine (ICG), et un logement de prévention de fuite de lumière (15) formé pour prévenir une transmission de lumière externe ;
une unité d'acquisition de données (30) incluant un filtre passe-bande (31) pour ne faire passer à travers celui-ci qu'une lumière ayant une longueur d'onde dans le proche infrarouge, dans une image de fluorescence émise par l'ICG et le dispositif d'émission de lumière, et une caméra (33) à dispositif de couplage de charge (CCD) pour photographier une région de longueur d'onde dans le proche infrarouge ;
une unité de traitement de données (50) pour calculer un taux de perfusion du corps vivant sur la base de données relatives à la région de longueur d'onde dans le proche infrarouge par l'intermédiaire d'un câble de données connecté à l'unité d'acquisition de données ; et
une unité de sortie de données (70) pour délivrer en sortie le taux de perfusion calculé par l'unité de traitement de données,
**caractérisé en ce que** le logement de prévention de fuite de lumière (15) inclut une unité de support pour les jambes (13), qui est une structure apte à supporter les jambes du corps vivant.

2. Appareil selon la revendication 1, dans lequel l'unité de support pour les jambes (13) inclut:

un support pour les jambes (13a) ayant une certaine largeur et une certaine hauteur, nécessaire pour supporter les deux jambes du corps vivant ; et
un repose-pieds (13b) mis en oeuvre pour permettre que les pieds du corps vivant soient placés sur celui-ci.

3. Appareil selon la revendication 1, dans lequel le dispositif d'émission de lumière (11) est mis en oeuvre en utilisant l'un quelconque d'un laser ayant une longueur d'onde de 750 à 780 nm, d'une source de lumière blanche, et d'une diode électroluminescente, chacun ayant un filtre passe-bande correspondant à une région de la longueur d'onde

4. Appareil selon la revendication 1, dans lequel le dispositif d'émission de lumière (11) est mis en oeuvre en utilisant un ou plusieurs dispositif(s) d'émission de lumière, et est construit de façon à être apte à ajuster une intensité de la lumière émise par celui-ci

5. Appareil selon la revendication 1, dans lequel l'unité de traitement de données (50) inclut:

un moyen de numérisation (51) pour traiter des données entrées par l'intermédiaire du câble de données pour avoir des intensités de fluorescence pour des régions respectives dans le temps dans une région d'intérêt ; un moyen de détermination (53) pour reconnaître des tracés d'ischémie de régions respectives sur la base des intensités de fluorescence dans le temps, et calculer des coefficients de corrélation entre les tracés d'ischémie et des régions adjacentes ; et un moyen de calcul de taux de perfusion (55) pour calculer le taux de perfusion en utilisant les données entrées du moyen de détermination

6. Appareil selon la revendication 5, dans lequel l'unité de sortie de données (70) attribue des couleurs prédéfinies en fonction du taux de perfusion et colorie des régions respectives dans les couleurs attribuées, délivrant ainsi visuellement en sortie le taux de perfusion sous la forme d'une image

EP 1 967 134 B1

FIG. 1

```
ICG INJECTION →  ┌─────────────┐      ┌──────────────────────────┐     ┌──────────────────────┐     ┌─────────────────────────────┐      ┌──────────────────────┐
                 │   LIGHT     │      │  LIGHT LEAKAGE           │     │  DATA ACQUISITION    │     │  DATA PROCESSING UNIT        │      │  DATA OUTPUT UNIT     │
                 │   EMIT-     │ ──►  │  PREVENTION UNIT         │ ──► │  UNIT                │ ──► │                              │ ──►  │                      │
                 │   TING      │      │                          │     │                      │     │                              │      │                      │
                 │   DEVICE    │      │  ┌────────────────────┐  │     │  ┌────────────┐      │     │ ┌────────┐ ┌────────┐ ┌────────┐ │    │  ┌────────────────┐  │
                 └─────────────┘      │  │ LEG SUPPORT UNIT   │  │     │  │ BAND       │      │     │ │DIGITIZ-│ │DETERM- │ │PERFUSION││    │  │ FLUORESCENCE   │  │
                      11              │  │ ┌──────┐ ┌───────┐ │  │     │  │ PASS       │      │     │ │ATION   │ │INATION │ │RATE     ││    │  │ IMAGE          │  │
                                      │  │ │ LEG  │ │FOOTREST│ │  │     │  │ FILTER     │      │     │ │MEANS   │ │MEANS   │ │CALCULA- ││    │  │                │  │
                                      │  │ │SUPPORT│ │        │ │  │     │  └────────────┘      │     │ └────────┘ │        │ │TION     ││    │  └────────────────┘  │
                                      │  │ └──────┘ └───────┘ │  │     │  ┌────────────┐      │     │            └────────┘ │MEANS    ││    │       71             │
                                      │  │  13a      13b       │  │     │  │ CCD        │      │     │   51        53       └────────┘ │    │                      │
                                      │  └────────────────────┘  │     │  │ CAMERA     │      │     │                        55       │    │                      │
                                      │         13                │     │  └────────────┘      │     │                                 │    │                      │
                                      │         15                │     │       33             │     │                                 │    │                      │
                                      └──────────────────────────┘     │       31             │     └─────────────────────────────┘      └──────────────────────┘
                                              10                        └──────────────────────┘              50                                  70
                                                                               30
                                        ↑1                     DATA CABLE
                                                                   40
```

10

## FIG. 2

FLUORESCENCE
IMAGE
OUTPUT

FIG. 3

FLUORESCENCE
IMAGE
OUTPUT

## FIG. 4A

FLUORESCENCE
IMAGE
OUTPUT

50

70

10

15

40

33

OPTICAL LENS

31

30

13
13b
13a

W

h

## FIG. 4B

FLUORESCENCE
IMAGE
OUTPUT

# FIG. 5

```
        ┌─────────────┐
        │   START     │
        └──────┬──────┘
               ▼
```

┌────────────────────────────────────────────────┐
│         INJECT ICG INTO LIVING BODY AND          │     S10
│    CONTINUOUSLY MEASURE ICG CONCENTRATION        │
│        UNTIL CONCENTRATION DECREASES             │
└────────────────────────────────────────────────┘
                       │
                       ▼
┌────────────────────────────────────────────────┐
│        SPATIALLY AND TEMPORALLY DIGITIZE         │     S20
│           ICG CONCENTRATION VARIATION            │
└────────────────────────────────────────────────┘
                       │
                       ▼
┌────────────────────────────────────────────────┐
│   RECOGNIZE ISCHEMIC PATTERNS FOR RESPECTIVE     │     S30
│   REGIONS ON THE BASIS OF DIGITIZED DATA AND     │
│       CALCULATE CORRELATION COEFFICIENTS         │
│ BETWEEN ISCHEMIC PATTERNS AND ADJACENT REGIONS   │
└────────────────────────────────────────────────┘
                       │
                       ▼
┌────────────────────────────────────────────────┐
│           CALCULATE PERFUSION RATE               │     S40
└────────────────────────────────────────────────┘
                       │
                       ▼
┌────────────────────────────────────────────────┐
│           OUTPUT FLUORESCENCE IMAGE              │     S50
└────────────────────────────────────────────────┘
                       │
                       ▼
        ┌─────────────┐
        │    END      │
        └─────────────┘

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10059070 C1

- WO 0122870 A